# EUROPÄISCHE PATENTSCHRIFT

(11) **EP 1 395 573 B1**
(45) Veröffentlichungstag und Bekanntmachung des Hinweises auf die Patenterteilung: **21.09.2005**
(21) Anmeldenummer: 02740603.2
(22) Anmeldetag: 15.05.2002
(51) Int. Cl.: C07D 401/04, C07M 7/00

(54) **VERFAHREN ZUR SPALTUNG DES METHYL 4-(2-CHLOR-4-FLUORPHENYL)-2-(3,5-DIFLUOR-2-PYRIDINYL)-6-METHYL-1,4-DIHYDRO-5-PYRIMIDINCARBOXYLAT-RACEMATS**
METHOD FOR SEPARATING METHYL 4-(2-CHLORO-4-FLUOROPHENYL)-2-(3,5-DIFLUORO-2-PYRIDINYL)-6-METHYL-1,4-DIHYDRO-5-PYRIMIDINE- CARBOXYLATE -RACEMATE
PROCEDE POUR LA SEPARATION DU RACEMIQUE DE METHYL 4-(2-CHLORO-4-FLUOROPHENYL)-2-(3,5-DIFLUORO-2-PYRIDINYL)-6-METHYL-1,4-DIHYDRO-5-PYRIMIDINE-CARBOXYLATE

(30) Priorität: 23.05.2001 DE 10125131
(43) Veröffentlichungstag der Anmeldung: 10.03.2004
(73) Patentinhaber: Bayer HealthCare AG, 51368 Leverkusen (DE)
(72) Erfinder: GOLDMANN, Siegfried, 42327 Wuppertal (DE); FEY, Peter, 42111 Wuppertal (DE)
(86) Internationale Anmeldenummer: PCT/EP2002/005339
(87) Internationale Veröffentlichungsnummer: WO 2002/094807

(56) Entgegenhaltungen:
- WO-A-00/58302

## Beschreibung

Die Erfindung betrifft ein Verfahren zur Trennung der enantiomeren Methyl 4-(2-chlor-4-fluorphenyl)-2-(3,5-difluor-2-pyridinyl)-6-methyl-1,4-dihydro-5-pyrimidin-carboxylate mit Hilfe von (-)-Camphansäure.

Die Verbindung Methyl 4-(2-chlor-4-fluorphenyl)-2-(3,5-difluor-2-pyridinyl)-6-methyl-1,4-dihydro-5-pyrimidincarboxylat entspricht der Formel

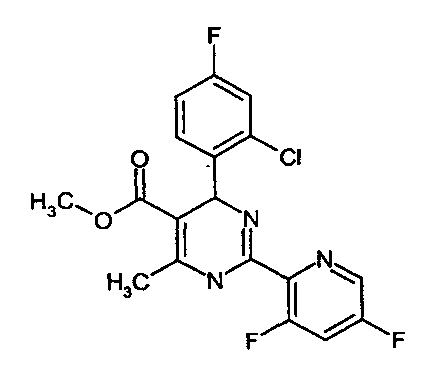

Sie kann als HBV-core-Protein-Inhibitor wirken und ist zur Prophylaxe und zur Behandlung von Hepatitis, insbesondere von Hepatitis B, geeignet. Die Verbindung und verschiedene Verfahren zu ihrer Herstellung sind aus der EP-A 1 080 086 (Beispiel 61) bekannt. Die bevorzugte optisch aktive Form ist das (-)-Enantiomere. Die Enantiomeren können an chiralen Säulen getrennt werden. Obwohl diese Methode zu guten Ergebnissen führt, ist sie fiir eine großtechnische Produktion wenig geeignet.

Es wurde nun gefunden, dass sich die beiden enantiomeren Methyl 4-(2-chlor-4-fluorphenyl)-2-(3,5-difluor-2-pyridinyl)-6-methyl-1,4-dihydro-5-pyrimidincarboxylate über ihre diastereomeren (-)-Camphansalze trennen lassen; das (-)-Camphansalz des (R)-Enantiomeren ist nämlich in vielen Lösungsmitteln schwerer löslich als das (-)-Camphansalz des (L)-Enantiomeren. Der Begriff "Salze" im Sinne dieser Erfindung umfasst auch Cokristallisate; laut NMR-Spektrum liegt die Base vornehmlich in nicht-protonierter Form vor.

Gegenstand der Erfindung ist daher ein Verfahren zur Trennung der enantiomeren Methyl 4-(2-chlor-4-fluorphenyl)-2-(3,5-difluor-2-pyridinyl)-6-methyl-1,4-dihydro-5-pyrimidincarboxylate mit Hilfe von (-)-Camphansäure.

Die unterschiedliche Löslichkeit der diastereomeren Salze kann man grundsätzlich für zwei verschiedene Arten ihrer Trennung nutzen: nämlich einerseits durch (fraktionierte) Kristallisation des schwerer löslichen Diastereomeren aus Lösung oder andererseits durch Herauslösen des leichter löslichen Diastereomeren aus dem Gemisch der festen Diastereomeren.

Man geht also in der Regel so vor, dass man (-)-Camphansäure und das zu trennende Racemat zu den entsprechenden diastereomeren Salzen umsetzt und entweder a) das Umsetzungsprodukt löst und durch Kühlen der Lösung und/oder teilweises Abziehen des Lösungsmittels das schwerer lösliche Produkt zum Ausfallen bringt und das ausgefallene Produkt von der Lösung abtrennt oder b) das feste Umsetzungsprodukt mit Lösungsmittel behandelt, um das leichter lösliche Produkt zu lösen und diese Lösung vom schwerer löslichen Rückstand abzutrennen.

Unter den Begriff "Lösungsmittel" im Sinne der Erfindung fallen alle gängigen Lösungsmittel, z.B. Kohlenwasserstoffe wie Benzol, Xylol, Toluol, Hexan, Cyclohexan oder Erdölfraktionen, Halogenkohlenwasserstoffe wie Dichlormethan, Trichlormethan, Tetrachlormethan, Trichlorethan, Tetrachlorethan, 1,2-Dichlorethan oder Trichlorethylen, Alkohole wie Methanol, Ethanol, n-Propanol, iso-Propanol, n-Butanol oder tert.-Butanol, Ether wie Diethylether, Methylisopropylether, Diisopropylether, Glykolmonomethylether, Glykoldimethylether, Diethylenglykoldimethylether, Tetrahydrofuran, Dioxan, Ketone wie Aceton, Methylethylketon, Carbonsäuren wie Eisessig, Ester wie Ethyl- und Butylacetat, Heterocyclen wie Pyridin oder aprotische Lösungsmittel wie Nitromethan, Acetonitril, Dimethylformamid, Dimethylsulfoxid und Hexamethylphosphorsäuretriamid und die Mischungen der genannten Verbindungen. Gegebenenfalls kann wassermischbaren organischen Lösungsmitteln auch Wasser zugesetzt werden. Bevorzugte Lösungsmittel für die Umsetzung des zu trennenden Racemats mit (-)-Camphansäure sind Alkohole, insbesondere Ethanol; bevorzugte Lösungsmittel für die Trennung der diastereomeren Salze sind Ether, Ester und Ketone, insbesondere Diisopropylether.

Das Lösen des Diastereomeren kann innerhalb eines weiten Temperaturbereichs erfolgen; vorzugsweise bei der Siedetemperatur des Lösungsmittels bis 20°C. Auch die Temperaturen bei der Kristallisation des schwerer löslichen diastereomerenreinen Salzes oder beim Auswaschen des leichter löslichen diastereomerenreinen Salzes kann innerhalb eines weiten Bereichs liegen; sie beträgt vorzugsweise -40 bis 20°C, bevorzugt um 0°C.

Die Freisetzung des freien Enantiomeren aus dem diastereomerenreinen Salz kann mit jeder üblichen Base wie Ammoniak, Alkalihydroxiden wie Natrium- oder Kaliumhydroxid, Alkalicarbonaten oder -hydrogencarbonaten wie Natrium- oder Kaliumcarbonat bzw. -hydrogencarbonat erfolgen. Aus der wäßrigen basischen Lösung kann man das reine Enantiomere dann ohne weiteres mit einem organischen Lösungsmittel extrahieren.

Die (-)-Camphansäure kann durch Ansäuern ihrer wässrigen Lösung gefällt und dann erneut verwendet werden.

Weiterer Gegenstand der Erfindung ist D- und L-Methyl 4-(2-chlor-4-fluorphenyl)-2-(3,5-difluor-2-pyridinyl)-6-methyl-1,4-dihydro-5-pyrimidincarboxylat-(-)-Camphansäuresalz.

### Beispiel: Racematspaltung mit (-)-Camphansäure

### (-)-Methyl (4R)-4-(2-chlor-4-fluorohenyl)-2-(3,5-difluor-2-pyridinyl)-6-methyl-1,4-dihydro-5-pyrimidincarboxylat

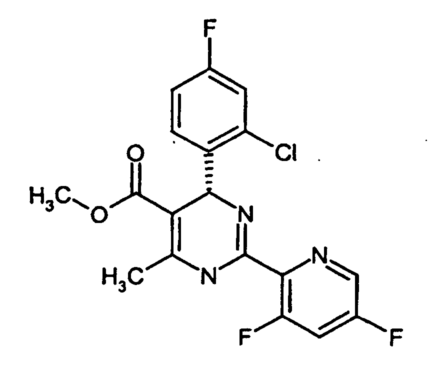

37,68 g (95,21 mmol) Methyl 4-(2-chlor-4-fluorphenyl)-2-(3,5-difluor-2-pyridinyl)-6-methyl-1,4-dihydro-5-pyrimidincarboxylat und 18,87 g (95,21 mmol) (-)-Camphansäure wurden in siedendem Ethanol gelöst. Nach dem Abkühlen wurde das Ethanol bis zur Trockne verdampft; der Rückstand wurde zerkleinert, mit Diisopropylether gemischt und auf Rückflusstemperatur erwärmt. Nach dem Abkühlen auf 0°C und Stehen über Nacht wurden die Kristalle abgesaugt und mit kaltem Diisopropylether gewaschen. Der Filterkuchen wurde in Ethylacetat suspendiert, mit 10 gew.-%iger wässriger Natriumcarbonatlösung alkalisch gestellt und zweimal mit Ethylacetat extrahiert. Die organischen Phasen wurden mit Natriumsulfat getrocknet und eingedampft. Der Rückstand wurde mit wenig kaltem Ethanol kristallisiert. Man erhielt 17,7 g (93,7 %) Methyl (-)-4-(2-chlor-4-fluorphenyl)-2-(3,5-difluor-2-pyridinyl)-6-methyl-1,4-dihydro-5-pyrimidincarboxylat (ee 97 %, HPLC).

Die absolute Konfiguration (R) wurde röntgenographisch bestimmt.

## Patentansprüche

1. Verfahren zur Spaltung der Enantiomeren von Methyl 4-(2-chlor-4-fluorphenyl)-2-(3,5-difluor-2-pyridinyl)-6-methyl-1,4-dihydro-5-pyrimidincarboxylat mit Hilfe von (-)-Camphansäure.

2. Verfahren nach Anspruch 1, wonach man die (-)-Camphansäuresalze des (D/L)-Methyl 4-(2-chlor-4-fluorphenyl)-2-(3,5-difluor-2-pyridinyl)-6-methyl-1,4-dihydro-5-pyrimidincarboxylats durch Kristallisation trennt und aus dem abgetrennten festen (-)-Camphansäuresalz und/oder aus der abgetrennten Lösung das oder die Enantiomeren mit einer Base freisetzt und gegebenenfalls isoliert.

3. Verfahren nach Anspruch 1, wonach man aus der Mischung der (-)-Camphansäuresalze des (D/L)-Methyl 4-(2-chlor-4-fluorphenyl)-2-(3,5-difluor-2-pyridinyl)-6-methyl-1,4-dihydro-5-pyrimidincarboxylats das leichter lösliche Diastereomer herauslöst, die entstandene Lösung vom schwerer löslichen Diastereomer trennt, aus dem abgetrennten festen (-)-Camphansäuresalz und/oder aus der abgetrennten Lösung das oder die Enantiomeren mit einer Base freisetzt und gegebenenfalls isoliert.

4. D-Methyl 4-(2-chlor-4-fluorphenyl)-2-(3,5-difluor-2-pyridinyl)-6-methyl-1,4-dihydro-5-pyrimidincarboxylat-(-)-Camphansäuresalz.

5. L-Methyl 4-(2-chlor-4-fluorphenyl)-2-(3,5-difluor-2-pyridinyl)-6-methyl-1,4-dihydro-5-pyrimidincarboxylat-(-)-Camphansäuresalz.

## Claims

1. Method for resolving the enantiomers of methyl 4-(2-chloro-4-fluorophenyl)-2-(3,5-difluoro-2-pyridinyl)-6-methyl-1,4-dihydro-5-pyrimidinecarboxylate with the aid of (-)-camphanic acid.

2. Method according to Claim 1, wherein the (-)-camphanic acid salts of (D/L)-methyl 4-(2-chloro-4-fluorophenyl)-2-(3,5-difluoro-2-pyridinyl)-6-methyl-1,4-dihydro-5-pyrimidinecarboxylate are separated by crystallization, and the enantiomer(s) is (are) liberated with a base, and isolated where appropriate, from the removed solid (-)-camphanic acid salt and/or from the removed solution.

3. Method according to Claim 1, wherein the more soluble diastereomer is dissolved out of the mixture of the (-)-camphanic acid salts of (D/L)-methyl 4-(2-chloro-4-fluorophenyl)-2-(3,5-difluoro-2-pyridinyl)-6-methyl-1,4-dihydro-5-pyrimidinecarboxylate, the resulting solution is separated from the less soluble diastereomer, and the enantiomer(s) is (are) liberated with a base, and isolated where appropriate, from the removed solid (-)-camphanic acid salt and/or from the removed solution.

4. D-Methyl 4-(2-chloro-4-fluorophenyl)-2-(3,5-difluoro-2-pyridinyl)-6-methyl-1,4-dihydro-5-pyrimidinecarboxylate (-)-camphanic acid salt.

5. L-Methyl 4-(2-chloro-4-fluorophenyl)-2-(3,5-difluoro-2-pyridinyl)-6-methyl-1,4-dihydro-5-pyrimidinecarboxylate (-)-camphanic acid salt.

## Revendications

1. Procédé de dédoublement des énantiomères du 4-(2-chloro-4-fluorophényl)-2-(3,5-difluoro-2-pyridinyl)-6-méthyl-1,4-dihydro-5-pyrimidinecarboxylate de méthyle à l'aide d'acide (-)camphanoïque.

2. Procédé suivant la revendication 1, dans lequel les sels d'acide (-)camphanoïque du (D/L)-4-(2-chloro-4-fluorophényl)-2-(3,5-difluoro-2-pyridinyl)-6-méthyl-1,4-dihydro-5-pyrimidinecarboxylate de méthyle sont séparés par cristallisation et l'énantiomère ou les énantiomères sont libérés avec une base du sel d'acide (-)camphanoïque solide séparé et/ou de la solution séparée et sont éventuellement isolés.

3. Procédé suivant la revendication 1, dans lequel on extrait par dissolution le diastéréoisomère le plus soluble du mélange des sels d'acide (-)camphanoïque du (D/L)-4-(2-chloro-4-fluorophényl)-2-(3,5-difluoro-2-pyridinyl)-6-méthyl-1,4-dihydro-5-pyrimidinecarboxylate de méthyle, on sépare la solution produite du diastéréoisomère le moins soluble, on libère avec une base l'énantiomère ou les énantiomères du sel solide d'acide (-)camphanoïque séparé et/ou de la solution séparée, et on les isole éventuellement.

4. Le sel d'acide (-)camphanoïque du D-4-(2-chloro-4-fluorophényl)-2-(3,5-difluoro-2-pyridinyl)-6-méthyl-1,4-dihydro-5-pyrimidinecarboxylate de méthyle.

5. Le sel d'acide (-)camphanoïque du L-4-(2-chloro-4-fluorophényl)-2-(3,5-difluoro-2-pyridinyl)-6-méthyl-1,4-dihydro-5-pyrimidinecarboxylate de méthyle.
